# EUROPEAN PATENT APPLICATION

(11) **EP 4 799 654 A1**
(43) Date of publication of application: **02.09.2026**
(21) Application number: 24861301.0
(22) Date of filing: 21.11.2024
(51) Int. Cl.: A61L 2/20, A61L 2/24

(54) **STERILIZATION DEVICE**

(30) Priority: 14.12.2023 KR 20230181504
(71) Applicant: Lowtem Co., Ltd., Gunpo-si, Gyeonggi-do 15850 (KR)
(72) Inventor: LEE, Sang Il, Anyang-si, Gyeonggi-do 13922 (KR)
(74) Representative: BCKIP Part mbB
(86) International application number: PCT/KR2024/018533
(87) International publication number: WO 2025/127480

(57) **Abstract**

The present invention relates to a sterilization device capable of sterilizing an object to be sterilized using hydrogen peroxide as a sterilant. A sterilization apparatus according to an embodiment of the present invention includes a sterilization housing having a sterilization chamber configured to receive an object to be sterilized, a vaporizer connected to the sterilization housing to supply hydrogen peroxide to the sterilization chamber, a supplier configured to supply a hydrogen peroxide solution to the vaporizer, a vacuum pump configured to apply a negative pressure to the vaporizer in order to lower an internal pressure of the vaporizer to an evaporation pressure enabling evaporation of moisture from the hydrogen peroxide solution, a connector assembly including a connecting flow path configured to interconnect the vaporizer and the vacuum pump and a connector valve configured to open and close the connecting flow path, and a controller configured to control the vacuum pump and the connector valve. As the time for which the negative pressure is applied to the vaporizer is varied in accordance with a predetermined condition, a concentration of the hydrogen peroxide solution supplied to the vaporizer may be varied.

## Description

### [Technical Field]

The present invention relates to a sterilization apparatus, and more particularly to a sterilization apparatus capable of sterilizing an object to be sterilized using hydrogen peroxide as a sterilant.

### [Background Art]

A sterilization apparatus is used to sterilize an object, to be sterilized, such as a medical device or the like. Such a sterilization apparatus is configured to completely remove all kinds of living microorganisms through physical and chemical action.

As sterilization methods of the sterilization apparatus, there are autoclave sterilization and chemical sterilization.

A sterilization apparatus using autoclave sterilization sterilizes an object, to be sterilized, using moist heat in a pressurized saturated vapor state. Such a sterilization apparatus is not suitable for sterilization of an object, to be sterilized, vulnerable to heat because the sterilization apparatus sterilizes the object at a high temperature.

A sterilization apparatus using chemical sterilization sterilizes an object, to be sterilized, using a chemical sterilant. As the chemical sterilant, there are hydrogen peroxide, peracetic acid, etc.

Hydrogen peroxide used for chemical sterilization has a rapid sterilization rate by virtue of a high sterilization effect thereof, and may secure safety of an object, to be sterilized, by virtue of low-temperature sterilization thereof. Since residuals are water and oxygen, hydrogen peroxide is safe and environmentally friendly to the user. Hydrogen peroxide is an oxidant-based sterilant and, as such, may disrupt membrane lipids of cell membranes, DNA, and other essential cell components, and may remove even spores of microorganisms which cannot be removed by a general disinfectant.

For effective sterilization, it is desirable to use a highly-concentrated hydrogen peroxide solution. However, when such a hydrogen peroxide solution has a predetermined concentration (for example, 60wt%) or more, there is a limitation in handling such as transportation, storage, etc. for safety reasons such as explosion or the like.

For this reason, in association with the sterilization apparatus using hydrogen peroxide, a low-concentration hydrogen peroxide solution is supplied, and is used in a state of being concentrated to a concentration required for sterilization.

Therefore, in order to improve the sterilization apparatus using hydrogen peroxide, technology capable of effectively concentrating a low-concentration hydrogen peroxide solution to an appropriate concentration required for sterilization is needed.

### [Disclosure]

### [Technical Problem]

Therefore, the present invention has been made in view of the above problems, and it is an object of the present invention to provide a sterilization apparatus capable of effectively concentrating a low-concentration hydrogen peroxide solution to a concentration required for sterilization and enhancing sterilization efficiency.

Objects of the present invention are not limited to the above-described object, and other objects of the present invention not yet described will be more clearly understood by those skilled in the art from the following detailed description.

### [Technical Solution]

In accordance with an embodiment of the present invention, the above and other objects can be accomplished by the provision of a sterilization apparatus including a sterilization housing having a sterilization chamber configured to receive an object to be sterilized, a vaporizer connected to the sterilization housing to supply hydrogen peroxide to the sterilization chamber, a supplier configured to supply a hydrogen peroxide solution to the vaporizer, a vacuum pump configured to apply a negative pressure to the vaporizer in order to lower an internal pressure of the vaporizer to an evaporation pressure enabling evaporation of moisture from the hydrogen peroxide solution, a connector assembly including a connecting flow path configured to interconnect the vaporizer and the vacuum pump and a connector valve configured to open and close the connecting flow path, and a controller configured to control the vacuum pump and the connector valve, wherein, as the time for which the negative pressure is applied to the vaporizer is varied in accordance with a predetermined condition, a concentration of the hydrogen peroxide solution supplied to the vaporizer is variable.

The connector assembly may include a first end connector configured to interconnect the vaporizer and the connector valve while having a first end connector flow path, a connector pipe disposed between the connector valve and the vacuum pump while having a connector pipe flow path, a first middle connector connected to one end of the connector pipe to interconnect the connector valve and the connector pipe while having a first middle connector flow path, a second middle connector connected to the other end of the connector pipe while having a second middle connector flow path, and a second end connector configured to interconnect the second middle connector and the vacuum pump while having a second end connector flow path.

The first end connector flow path, a valve flow path of the connector valve, the first middle connector flow path, the connector pipe flow path, the second middle connector flow path, and the second end connector flow path may form the connecting flow path. The connecting flow path may have at least one diameter-increased section and at least one diameter-decreased section.

The first end connector flow path may be a smallest-diameter portion of the connecting flow path.

The dimeter of the first middle connector flow path may be equal to the diameter of the second middle connector flow path. The diameter of the connector pipe flow path may be greater than the diameter of the first middle connector flow path and the diameter of the second middle connector flow path.

The second end connector may include a first connection portion connected to the second middle connector, a second connection portion connected to the vacuum pump, and a third connection portion connected to the sterilization housing.

The time for which the negative pressure is applied to the vaporizer may be varied to correspond to multiples of a predetermined minimum reference time.

In the minimum reference time, an output of the vacuum pump may be controlled such that the internal pressure of the vaporizer is linearly lowered at a lowering rate according to a predetermined reference slope within a range not exceeding a predetermined first target pressure.

When the time for which the negative pressure is applied to the vaporizer increases to correspond to n times the predetermined minimum reference time, an output of the vacuum pump may be controlled such that the internal pressure of the vaporizer is linearly lowered at a lowering rate according to the reference slope in the minimum reference time and reaches the predetermined first target pressure when the minimum reference time ends and such that the internal pressure of the vaporizer is linearly lowered at a lowering rate according to a predetermined slope after the minimum reference time ends and reaches a predetermined second target pressure when a time corresponding to n times the minimum reference time ends.

The sterilization apparatus according to the present invention may include a bypass pipe having a bypass pipe flow path connected to the connecting flow path, and a bypass valve controlled by the controller to open and close the bypass pipe flow path. When the internal pressure of the vaporizer is lowered at a lowering rate according to a greater slope than the reference slope in the minimum referent time, the bypass valve may be controlled such that fluid is introduced into the connecting flow path through the bypass pipe, in order to adjust a lowering rate of the internal pressure of the vaporizer. When the internal pressure of the vaporizer is lowered at a lowering rate according to a greater slope than the predetermined slope in the time corresponding to n times the minimum reference time, the bypass valve may be controlled such that fluid is introduced into the connecting flow path through the bypass valve, in order to adjust the lowering rate of the internal pressure of the vaporizer.

The bypass pipe may interconnect the sterilization housing and the connector assembly in order to enable gas from the sterilization chamber to flow to the connecting flow path.

### [Advantageous effects]

The sterilization apparatus according to the present invention as described above may enhance a sterilization effect for an object, to be sterilized, received in the sterilization chamber by concentrating hydrogen peroxide through evaporation of moisture from a hydrogen peroxide solution supplied to the vaporizer, and supplying the concentrated hydrogen peroxide to the sterilization chamber.

In addition, the sterilization apparatus according to the present invention may receive and use a low-concentration hydrogen peroxide solution because the hydrogen peroxide solution can be concentrated using the vaporizer. In addition, since the low-concentration hydrogen peroxide solution, which exhibits relatively low transportation and storage costs while exhibiting relatively easy handling, is usable, operating costs may be low.

Furthermore, the sterilization apparatus according to the present invention may concentrate the hydrogen peroxide solution to more precise and various concentrations to sterilize an object to be sterilized. For example, for an object, to be sterilized, having relatively high chemical resistance, the hydrogen peroxide solution may be concentrated to a relatively high concentration to perform sterilization and, as such, sterilization efficiency may be enhanced. On the other hand, for an object, to be sterilized, having relatively weak chemical resistance, the hydrogen peroxide solution may be concentrated to a relatively low concentration to perform sterilization and, as such, it may be possible to reduce damage to the object and to prevent a problem of a reduction in the lifespan of the object.

Effects of the present invention are not limited to the above-described effects. Other effects not described may be readily understood by those skilled in the art from the following description.

### [Description of Drawings]

FIGs. 1 and 2 are perspective views showing a sterilization apparatus according to an embodiment of the present invention.
FIG. 3 is a side view showing the sterilization apparatus according to the embodiment of the present invention.
FIG. 4 is a front view showing the sterilization apparatus according to the embodiment of the present invention.
FIG. 5 is a block diagram showing a part of configurations of the sterilization apparatus according to the embodiment of the present invention.
FIG. 6 is a sectional view showing a sterilization housing of the sterilization apparatus according to the embodiment of the present invention.
FIG. 7 is a perspective view showing a vaporizer and a connector assembly of the sterilization apparatus according to the embodiment of the present invention.
FIG. 8 is a sectional view showing the vaporizer and the connector assembly of the sterilization apparatus according to the embodiment of the present invention.
FIG. 9 is a graph depicting an example of a variation in internal pressure of the vaporizer in a procedure of concentrating a hydrogen peroxide solution in the sterilization apparatus according to the embodiment of the present invention.
FIG. 10 is a front view showing a sterilization apparatus according to another embodiment of the present invention.
FIG. 11 is a block diagram showing a part of configurations of the sterilization apparatus according to the other embodiment of the present invention.
FIG. 12 is a sectional view showing the vaporizer and the connector assembly in the sterilization apparatus according to the other embodiment of the present invention.
FIG. 13 is a graph depicting an example of a variation in internal pressure of the vaporizer in the procedure of concentrating the hydrogen peroxide solution in the sterilization apparatus according to the other embodiment of the present invention.

### [Description of Reference Numerals to Essential Parts]

100, 200: sterilization apparatus 110: sterilization housing
111: sterilization chamber 118: blower
122: introduction pipe 124: discharge pipe
126: introduction valve 128: discharge valve
130: supplier 132: supply pipe
140: vaporizer 150: vacuum pump
160: connector assembly 161; connecting flow path
162: first end connector 165: connector valve
170: connector pipe 173: first middle connector
176: second middle connector 179: second end connector
190: controller 210: bypass pipe
213: bypass valve

### [Best Mode]

Hereinafter, embodiments of the present invention will be described in detail in order to enable those of ordinary skill in the art to which the invention pertains to easily implement the present invention. The present invention may be implemented in various different forms and is not limited to embodiments described herein.

In explaining the present invention, the sizes and shapes of constituent elements shown in the drawings may be exaggeratedly or simply illustrated for clarity and convenience of explanation.

In addition, terms specifically defined herein in consideration of configurations and functions of the present invention may be changed in accordance with the intention of a user or an operator or customary usages. Such items should be interpreted as having meanings and concepts that align with the technical concept of the present invention, as detailed in the specification.

Matters having no concern with the technical idea of the present invention will be omitted, for clarity of explanation of the present invention. The same or similar elements throughout the specification are designated by the same reference numerals.

In association with various embodiments, only a representative embodiment will be described under the condition that the same reference numbers are used to refer to the same parts. In the embodiments other than the representative embodiments, only configurations different from that of the representative embodiment will be described.

In the case where an element is described as being "connected" to another element throughout the specification, it should be understood that the element may be "directly connected" to the other element, or may be "indirectly connected" to the other element under the condition that another element is present therebetween. In addition, in the case where a part "includes" a constituent element, the term "includes" does not exclude addition of another constituent element, unless noted otherwise.

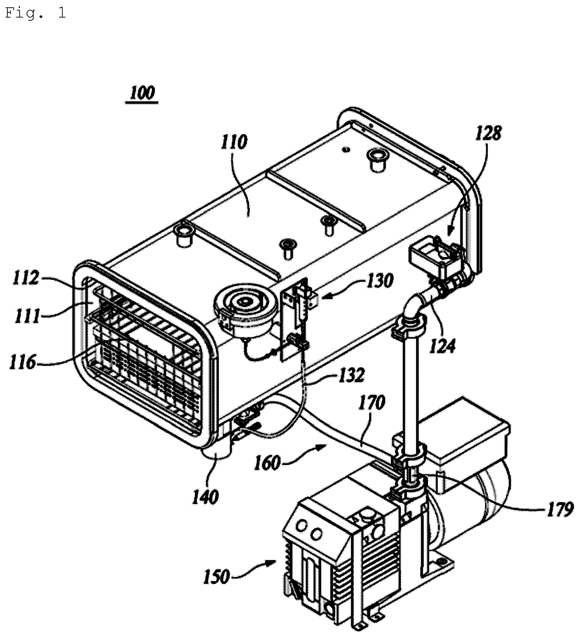

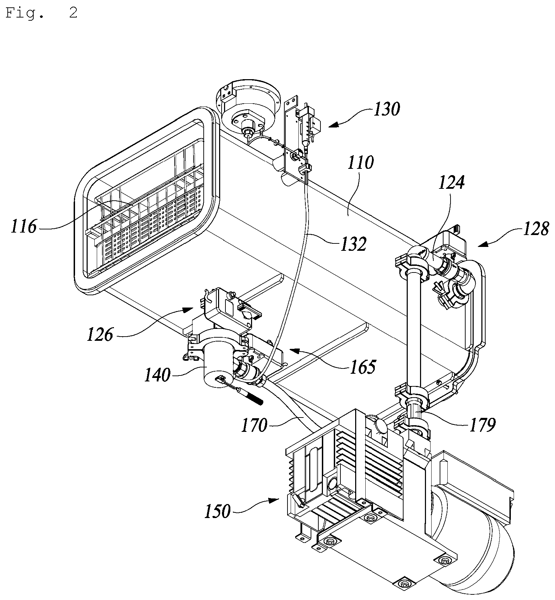

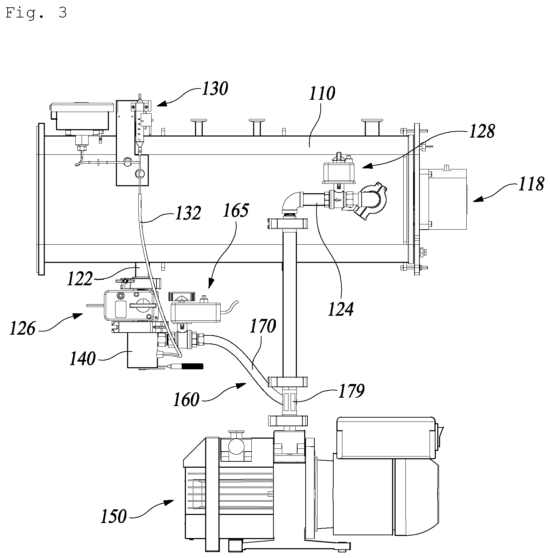

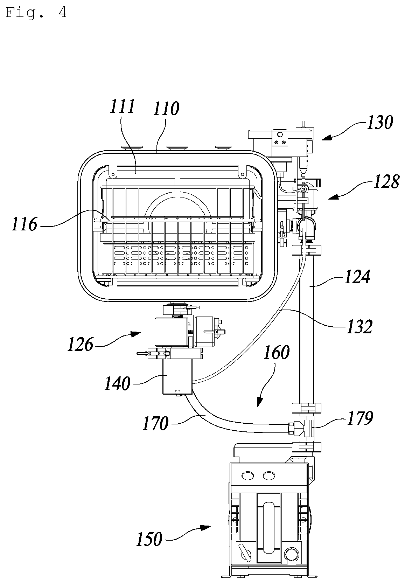

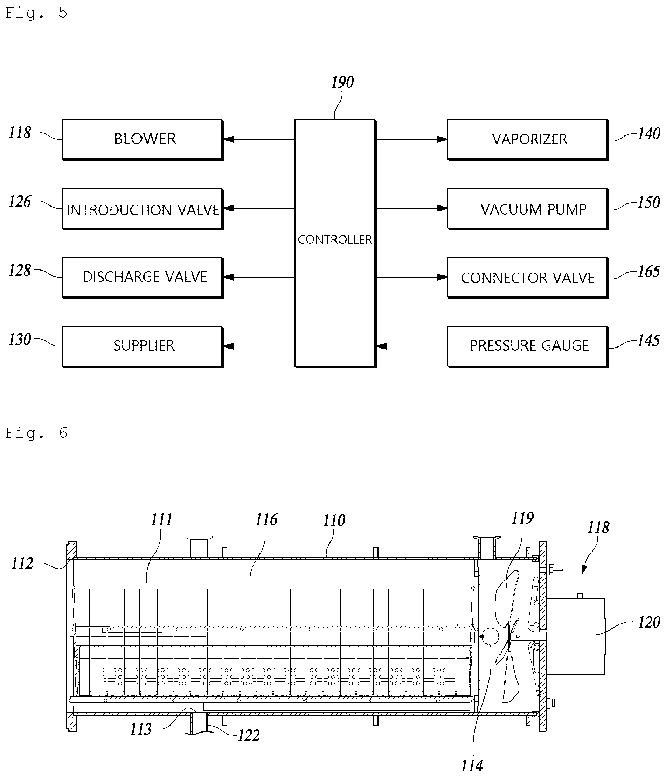

FIGs. 1 and 2 are perspective views showing a sterilization apparatus according to an embodiment of the present invention. FIG. 3 is a side view showing the sterilization apparatus according to the embodiment of the present invention. FIG. 4 is a front view showing the sterilization apparatus according to the embodiment of the present invention. FIG. 5 is a block diagram showing a part of configurations of the sterilization apparatus according to the embodiment of the present invention.

As shown in the drawings, a sterilization apparatus 100 according to an embodiment of the present invention includes a sterilization housing 110 configured to receive an object to be sterilized, a supplier 130 configured to supply a hydrogen peroxide solution, a vaporizer 140 configured to concentrate a hydrogen peroxide solution and to supply hydrogen peroxide to the sterilization housing 110, a vacuum pump 150 configured to supply a negative pressure to the vaporizer 140, a connection assembly 160 configured to interconnect the vaporizer 140 and the vacuum pump 150, and a controller 190 configured to control the vacuum pump 150, etc. The sterilization apparatus 100 according to the embodiment of the present invention receives a low-concentration hydrogen peroxide solution, concentrates the received low-concentration hydrogen peroxide solution to a concentration suitable for sterilization, and introduces concentrated hydrogen peroxide into the sterilization housing 110, thereby sterilizing an object, to be sterilized, in the sterilization housing 110.

Referring to FIGs. 1 to 4 and FIG. 6, a sterilization chamber 111, which is configured to receive an object to be sterilized, is provided within the sterilization housing 110. A tray 116 configured to support an object to be sterilized is disposed in the sterilization chamber 111. The sterilization housing 110 has an access port 112 configured to enable access of an object, to be sterilized, an inlet 113 configured to enable introduction of hydrogen peroxide, and an outlet 114 configured to enable discharge of fluid present in the sterilization chamber 111, such as air, hydrogen peroxide, etc. A door (not shown) configured to open and close the access port 112 may be installed at the sterilization housing 110.

The sterilization housing 110 is connected to the vaporizer 140 through an introduction pipe 122. The introduction pipe 122 is coupled to the sterilization housing 110 such that a flow path therein is connected to the inlet 113. Hydrogen peroxide concentrated in the vaporizer 140 may be introduced, in the form of vapors, into the sterilization chamber 111 through the introduction pipe 122 and the inlet 113.

An introduction valve 126 is connected to the introduction pipe 122. The introduction valve 126 opens and closes the flow path in the introduction pipe 122 and, as such, may allow and prevent flow of hydrogen peroxide through the introduction pipe 122. The introduction valve 126 may be configured to prevent flow of hydrogen peroxide through the introduction pipe 122 or to adjust a flow rate of hydrogen peroxide through the introduction pipe 122. In addition, the introduction valve 126 may be configured to have an automatic control valve structure automatically controlled by the controller 190.

The sterilization housing 110 is connected to the vacuum pump 150 through a discharge pipe 124. The discharge pipe 124 is connected to the sterilization housing 110 such that a flow path therein is connected to the outlet 114. When the vacuum pump 150 operates, fluid present in the sterilization chamber 111, such as air, hydrogen peroxide, etc., may be discharged through the outlet 114 and the discharge pipe 124.

A discharge valve 128 is connected to the discharge pipe 124. The discharge valve 128 opens and closes the flow path in the discharge pipe 124 and, as such, may allow and prevent flow of fluid through the discharge pipe 124. The discharge valve 128 may be configured to prevent flow of fluid through the discharge pipe 124 or to adjust a flow rate of fluid through the discharge pipe 124. In addition, the discharge valve 128 may be configured to have an automatic control valve structure automatically controlled by the controller 190.

A concrete configuration of the sterilization housing 110 is not limited to the shown configuration, and may be changed into other various configurations capable of receiving an object to be sterilized.

A blower 118 is installed at the sterilization housing 110. The blower 118 actively moves air or hydrogen peroxide in the sterilization chamber 111, thereby enhancing sterilization efficiency for an object, to be sterilized, by hydrogen peroxide. The blower 118 may include a fan 119 disposed in the sterilization chamber 111, and a motor 120 disposed outside the sterilization housing 110 and configured to provide rotational force to the fan 110. In addition, the blower 118 may be changed into other various configurations capable of generating a flow of fluid in the sterilization chamber 111.

In addition, a heater (not shown) configured to heat air or hydrogen peroxide present in the sterilization chamber 111, a thermometer (not shown) configured to measure a temperature of the sterilization chamber 111, and a pressure gauge (not shown) configured to measure a pressure of the sterilization chamber 111 may be installed at the sterilization housing 110.

The supplier 130 is intended to supply a hydrogen peroxide solution to the vaporizer 140. The supplier 130 may be connected to the vaporizer 140 through a supply pipe 132. The supplier 130 may be configured to automatically supply a predetermined amount of the hydrogen peroxide solution to the vaporizer 140 under control of the controller 190. A hydrogen peroxide solution diluted to a suitable concentration may be supplied to the vaporizer 140 through the supplier 130.

For example, a hydrogen peroxide solution diluted to a concentration of less than 40wt% for easy transportation or handling thereof may be supplied to the vaporizer 140 through the supplier 130. The hydrogen peroxide solution having a concentration of less than 40wt% is easy to store and handle and has low institutional constraints in association with dangerous cargo and, as such, has an advantage in that costs for transportation or storage may be reduced.

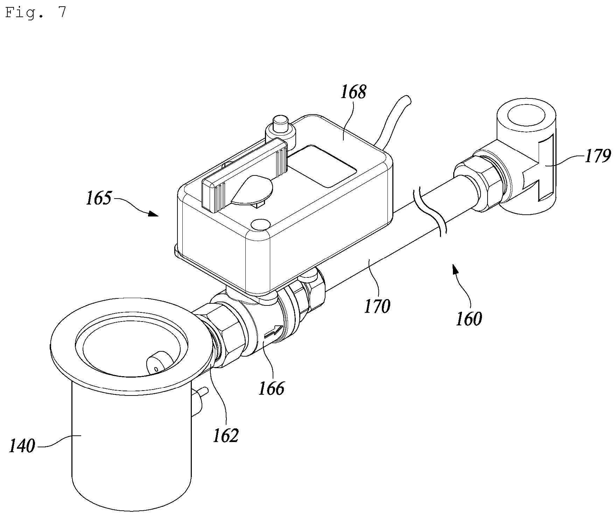

Referring to FIGs. 1 to 5 and FIGs. 7 and 8, the vaporizer 140 is connected to the sterilization housing 110 through the introduction pipe 122 in order to supply hydrogen peroxide to the sterilization chamber 111. The vaporizer 140 has an inner space configured to receive a hydrogen peroxide solution and to evaporate moisture or hydrogen peroxide from the hydrogen peroxide solution. A predetermined amount of the hydrogen peroxide solution may be supplied to the inner space of the vaporizer 140.

The volume of the inner space of the vaporizer 140 is greater than the volume of the hydrogen peroxide solution supplied to the vaporizer 140. When the hydrogen peroxide solution is supplied to the inner space of the vaporizer 140, the level of the supplied hydrogen peroxide solution in the inner space of the vaporizer 140 is lower than the height of the inner space of the vaporizer 140 and, as such, a space capable of accommodating moisture or hydrogen peroxide evaporated from the hydrogen peroxide solution may be secured over a solution surface of the hydrogen peroxide solution. The vaporizer 140 may concentrate the hydrogen peroxide solution supplied from the supplier 130 and may supply concentrated hydrogen peroxide in the form of vapors to the sterilization chamber 111.

The vaporizer 140 may concentrate the hydrogen peroxide solution by utilizing a vapor pressure difference between hydrogen peroxide and water. That is, when the internal pressure of the vaporizer 140 is lowered by the vacuum pump 150, moisture is evaporated from the hydrogen peroxide solution supplied to the vaporizer 140 and, as such, the hydrogen peroxide solution may be concentrated. Water is more rapidly evaporated because the vapor pressure thereof is higher than that of hydrogen peroxide, and is more rapidly diffused in a vapor phase than hydrogen peroxide because the molecular weight thereof is lower than that of hydrogen peroxide, and, as such, may be discharged from the vaporizer 140.

A pressure gauge 145 configured to measure an internal pressure of the vaporizer 140 may be installed at the vaporizer 140. A measurement signal from the pressure gauge 145 may be transmitted to the controller 190. The measurement signal from the pressure gauge 145 may be used to enable the controller 190 to control the vacuum pump 150.

In addition, a heater (not shown) configured to heat the hydrogen peroxide solution supplied to the vaporizer 140 and a thermometer (not shown) configured to measure an inner temperature of the vaporizer 140 may be installed at the vaporizer 140. A measurement signal from the thermometer may be used to enable the controller 190 to control the heater.

The vacuum pump 150 applies a negative pressure to the vaporizer 140 in order to lower the internal pressure of the vaporizer 140 to an evaporation pressure at which evaporation of moisture from the hydrogen peroxide solution is possible. The vacuum pump 150 is connected to the vaporizer 140 through the connector assembly 160. As moisture is removed from the hydrogen peroxide solution through operation of the vacuum pump 150, the hydrogen peroxide solution supplied to the vaporizer 140 may be concentrated.

In addition, the vacuum pump 150 is connected to the sterilization housing 110 through the discharge pipe 124. The vacuum pump 150 may form a vacuum atmosphere in the sterilization chamber 111 by applying a negative pressure to the sterilization housing 110.

The vacuum pump 150 may be controlled by the controller 190 to lower the internal pressure of the vaporizer 140 to a predetermined pressure or to lower the pressure of the sterilization chamber 111 to a predetermined pressure.

Referring to FIGs. 1 to 4 and FIGs. 7 and 8, the connector assembly 160 may interconnect the vaporizer 140 and the vacuum pump 150, and may provide a passage through which air, moisture, etc. present in the vaporizer 140 are discharged. The connector assembly 160 has a connecting flow path 161 functioning as a discharge passage of fluid. The connector assembly 160 includes a first end connector 162 connected to the vaporizer 140, a connector valve 165 configured to open and close the connecting flow path 161, a connector pipe 170 disposed between the connector valve 165 and the vacuum pump 150, a first middle connector 173 connected to one end of the connector pipe 170, a second middle connector 176 connected to the other end of the connector pipe 170, and a second end connector 179 connected to the vacuum pump 150.

The first end connector 162 interconnects the vaporizer 140 and the connector valve 165. A first end connector flow path 163 constituting the connecting flow path 161 is provided within the first end connector 162. The first end connector flow path 163 is a smallest-diameter portion of the connecting flow path 161. The first end connector flow path 163 is connected to a portion of the inner space of the vaporizer 140 disposed over the solution surface of the hydrogen peroxide solution supplied to the vaporizer 140. Accordingly, moisture evaporated from the hydrogen peroxide solution may be discharged through the first end connector flow path 163.

The connector valve 165 interconnects the first end connector 162 and the first middle connector 173. The connector valve 165 includes a valve body 166 formed with a valve flow path 167, and an opening/closing device 168 configured to open and close the valve flow path 167. The valve flow path 167 constitutes the connecting flow path 161. The connecting valve 165 may open and close the connecting flow path 161. That is, the opening/closing device 168 may allow or prevent flow of fluid through the connecting flow path 161 by opening or closing the valve flow path 167. The connector valve 165 may be controlled by the controller 190.

The connector pipe 170 is disposed between the connector valve 165 and the second end connector 179. The first middle connector 173 is connected to one end of the connector pipe 170, and the second middle connector 176 is connected to the other end of the connector pipe 170. A connector pipe flow path 171 constituting the connecting flow path 161 is provided within the connector pipe 170. The connector pipe 170 may be configured to have various shapes such as a bent pipe shape, a straight pipe shape, etc. in accordance with disposition of the connector valve 165 and the second end connector 179.

The first middle connector 173 interconnects the connector valve 165 and the connector pipe 170. A first middle connector flow path 174 constituting the connecting flow path 161 is provided within the first middle connector 173. The diameter of the first middle connector flow path 174 is greater than the diameter of the first end connector flow path 163 while being smaller than the diameter of the valve flow path 167 and the diameter of the connector pipe flow path 171.

The second middle connector 176 interconnects the connector pipe 170 and the second end connector 179. A second middle connector flow path 177 constituting the connecting flow path 161 is provided within the second middle connector 176. A concrete configuration of the second middle connector 176 is identical to that of the first middle connector 173, and the diameter of the second middle connector flow path 177 is equal to the diameter of the first middle connector flow path 174.

The second end connector 179 interconnects the second middle connector 176 and the vacuum pump 150. In addition, the second end connector 179 interconnects the discharge pipe 124 and the vacuum pump 150. A second end connector flow path 180 constituting the connecting flow path 161 is provided within the second end connector 179. The second end connector 179 includes a first connection portion 181 connected to the second middle connector 176, a second connection portion 182 connected to the vacuum pump 150, and a third connection portion 183 connected to the sterilization housing 110 through the discharge pipe 124.

The connecting flow path 161 of the connector assembly 160 includes a first end connector flow path 163, a valve flow path 167, a first middle connector flow path 174, a connector pipe flow path 171, a second middle connector flow path 177, and a second end connector flow path 180. Fluid emerging from the vaporizer 140 may flow to the vacuum pump 150 after sequentially passing through the first end connector flow path 163, the valve flow path 167, the first middle connector flow path 174, the connector pipe flow path 171, the second middle connector flow path 177, and the second end connector flow path 180.

The connecting flow path 161 has at least one diameter-increased section and at least one diameter-decreased section in a flow direction of fluid. Since fluid emerging from the first end connector flow path 163 is introduced into the valve flow path 167 and the diameter of the valve flow path 167 is greater than the diameter of the first end connector flow path 163, the valve flow path 167 may form a diameter-increased section. In addition, since fluid emerging from the valve flow path 167 is introduced into the first middle connector flow path 174 and the diameter of the first middle connector flow path 174 is smaller than the diameter of the valve flow path 167, the first middle connector flow path 174 may form a diameter-decreased section. In addition, since fluid emerging from the first middle connector flow path 174 is introduced into the connector pipe flow path 171 and the diameter of the connector pipe flow path 171 is greater than the diameter of the first middle connector flow path 174, the connector pipe flow path 171 may form a diameter-increased section. In addition, since fluid emerging from the connector pipe flow path 171 is introduced into the second middle connector flow path 177 and the diameter of the second middle connector flow path 177 is smaller than the diameter of the connector pipe flow path 171, the second middle connector flow path 177 may form a diameter-decreased section. In addition, since fluid emerging from the second middle connector flow path 177 is introduced into the second end connector flow path 180 and the diameter of the second end connector flow path 180 is greater than the diameter of the second middle connector flow path 177, the second end connector flow path 180 may form a diameter-increased section.

The connecting flow path 161 having at least one diameter-increased section and at least one diameter-decreased section in the flow direction of fluid, as described above, may discharge fluid from the vaporizer 140 at a suitable velocity. That is, when the vacuum pump 150 operates, the fluid introduced from the vaporizer 140 into the connecting flow path 161 may pass through the connecting flow path 161 at a flow velocity not excessively high or not excessively low because the fluid flows slowly in the diameter-increased section while flowing rapidly in the diameter-decreased section. The flow velocity of the fluid discharged from the vaporizer 140 through the connecting flow path 161 may mean a negative pressure application velocity or an internal pressure lowering velocity of the vaporizer 140. In addition, the negative pressure application velocity for the vaporizer 140 or the internal pressure lowering velocity of the vaporizer 140 is associated with an evaporation velocity of the hydrogen peroxide solution in the vaporizer 140. It may be possible to more precisely adjust a moisture evaporation velocity while suppressing evaporation of hydrogen peroxide by adjusting the negative pressure application velocity for the vaporizer 140 while lowering the internal pressure of the vaporizer 140 to an evaporation pressure enabling evaporation of moisture from the hydrogen peroxide solution, taking into consideration the above-described conditions. In addition, it may be possible to more precisely and diversely vary the concentration of the hydrogen peroxide solution by more precisely adjusting the moisture evaporation velocity of the hydrogen peroxide solution.

In the connecting flow path 161, the first end connector flow path 163 connected to the vaporizer 140 has the smallest diameter. Since the first end connector flow path 163 is connected to a portion of the inner space of the vaporizer 140 disposed over the solution surface of the hydrogen peroxide solution supplied to the vaporizer 140, the first end connector flow path 163 may more rapidly discharge moisture evaporated earlier than hydrogen peroxide and then moved to a space disposed over the hydrogen peroxide. Accordingly, discharge of hydrogen peroxide through the connecting flow path 161 may be minimized.

The controller 190 may control the blower 118, the introduction valve 126, the discharge valve 128, the supplier 130, the vacuum pump 150, and the connector valve 165. When the hydrogen peroxide solution supplied to the vaporizer 140 is to be concentrated, the controller 190 controls the introduction valve 126 and the discharge valve 128 to be closed such that both the flow path of the introduction pipe 122 and the flow path of the discharge pipe 124 are closed and the connecting flow path 161 is opened. In addition, when hydrogen peroxide concentrated in the vaporizer 140 is to be introduced into the sterilization chamber 111 to sterilize an object, to be sterilized, loaded in the sterilization chamber 111, the controller 190 may control the connector valve 165 to be closed and the introduction valve 126 to be opened such that the flow path of the introduction pipe 122 is opened and the connecting flow path 161 is closed. In addition, when residual gas is to be discharged from the sterilization chamber 111 after completion of sterilization of the object in the sterilization chamber 111, the controller 190 may control the introduction valve 126 to be closed and the discharge valve 128 to be opened such that the flow path of the introduction pipe 122 is closed and the flow path of the discharge pipe 124 is opened.

The sterilization apparatus 100 according to the embodiment of the present invention may more precisely and diversely vary the concentration of the hydrogen peroxide solution supplied to the vaporizer 140 through a method of optimizing a moisture evaporation velocity and a method of varying the time, for which a negative pressure is applied to the vaporizer 140, in accordance with a predetermined condition, using the above-described connector assembly 160.

The time for which the negative pressure is applied to the vaporizer 140 may be adjusted through operation of the connector valve 165. That is, it may be possible to vary the time, for which the negative pressure is applied to the vaporizer 140, by varying a point of time when the controller 190 controls the connector valve 165 to be closed in accordance with a predetermined process, during application of the negative pressure to the vaporizer 140 through operation of the vacuum pump 150. As the time for which the negative pressure is applied to the vaporizer 140 increases, the time for which moisture is discharged from the vaporizer 140 is increased and, as such, the hydrogen peroxide solution may be concentrated to a higher concentration.

Although the time for which the negative pressure is applied to the vaporizer 140 is varied in accordance with a predetermined condition during a procedure of concentrating the hydrogen peroxide solution, the output of the vacuum pump 150 may be constantly maintained. In this case, the internal pressure of the vaporizer 140 may be lowered at a constant rate in proportion to time.

The following Table 1 shows result values of an experimental example obtained after measurement of a concentration variation of a hydrogen peroxide solution according to a variation in the time for which a negative pressure is applied to the vaporizer.

**<Table 1>**

| Items | Time | | | | | |
|---|---|---|---|---|---|---|
| | 30 Sec. | 60 Sec. | 90 Sec. | 120 Sec. | 150 Sec. | 180 Sec. |
| Amount of Supplied Hydrogen Peroxide Solution | 10ml | 10ml | 10ml | 10ml | 10ml | 10ml |
| Concentration of Supplied Hydrogen Peroxide | 35w% | 35w% | 35w% | 35w% | 35w% | 35w% |
| Amount of Concentrated Hydrogen Peroxide Solution | 8.2ml | 7.0ml | 6.0ml | 4.9ml | 4.0ml | 3.2ml |
| Concentration of Concentrated Hydrogen Peroxide | 38w% | 42w% | 47w% | 52w% | 58w% | 63w% |

In the above experimental example, a sterilization apparatus manufactured to apply features of the present invention thereto was used, 10 ml of a hydrogen peroxide solution having a concentration of 35wt% was supplied to a vaporizer, and a concentration of the concentrated hydrogen peroxide solution was measured under a condition that the time for which a negative pressure is applied to the vaporizer is sequentially increased to correspond to multiples of a minimum reference time (30 seconds).

Referring to the above Table 1, it can be seen that, as the time for which the negative pressure is applied to the vaporizer increases, the volume of the hydrogen peroxide solution is reduced and, as such, the concentration of the hydrogen peroxide solution is increased.

In addition, referring to the above Table 1, it can be seen that, as the time for which the negative pressure is applied to the vaporizer increases to correspond to each multiple of the minimum reference time, the amount of the hydrogen peroxide solution is reduced at a reduction rate substantially similar to the increase rate of the negative pressure application time.

As described above, the sterilization apparatus 100 according to the embodiment of the present invention may enhance a sterilization effect for an object, to be sterilized, received in the sterilization chamber 111 by concentrating hydrogen peroxide through evaporation of moisture from a hydrogen peroxide solution supplied to the vaporizer 140, and supplying the concentrated hydrogen peroxide to the sterilization chamber 111.

In addition, the sterilization apparatus 100 according to the embodiment of the present invention may receive and use a low-concentration hydrogen peroxide solution because the hydrogen peroxide solution can be concentrated using the vaporizer 140. In addition, since the low-concentration hydrogen peroxide solution, which exhibits relatively low transportation and storage costs while exhibiting relatively easy handling, is usable, operating costs may be low.

Furthermore, the sterilization apparatus 100 according to the embodiment of the present invention may concentrate the hydrogen peroxide solution to more precise and various concentrations to sterilize an object to be sterilized. For example, for an object, to be sterilized, having relatively high chemical resistance, the hydrogen peroxide solution may be concentrated to a relatively high concentration to perform sterilization and, as such, sterilization efficiency may be enhanced. On the other hand, for an object, to be sterilized, having relatively weak chemical resistance, the hydrogen peroxide solution may be concentrated to a relatively low concentration to perform sterilization and, as such, it may be possible to reduce damage to the object and to prevent a problem of a reduction in the lifespan of the object.

Although the sterilization apparatus 100 according to the present invention has been described as varying the time, for which the negative pressure is applied to the vaporizer 140, through the method of controlling operation of the connector valve 165 in the procedure of concentrating the hydrogen peroxide solution, the sterilization apparatus 100 according to the present invention may vary the time, for which the negative pressure is applied to the vaporizer 140, through a method of controlling the vacuum pump 150.

In addition, although the sterilization apparatus 100 according to the present invention has been described as lowering the internal pressure of the vaporizer 140 at a constant rate in proportion to time in the procedure of concentrating the hydrogen peroxide solution, the sterilization apparatus 100 according to the present invention may operate such that the lowering rate of the internal pressure of the vaporizer 140 is varied in accordance with the time for which the negative pressure is applied to the vaporizer 140 in the procedure of concentrating the hydrogen peroxide solution.

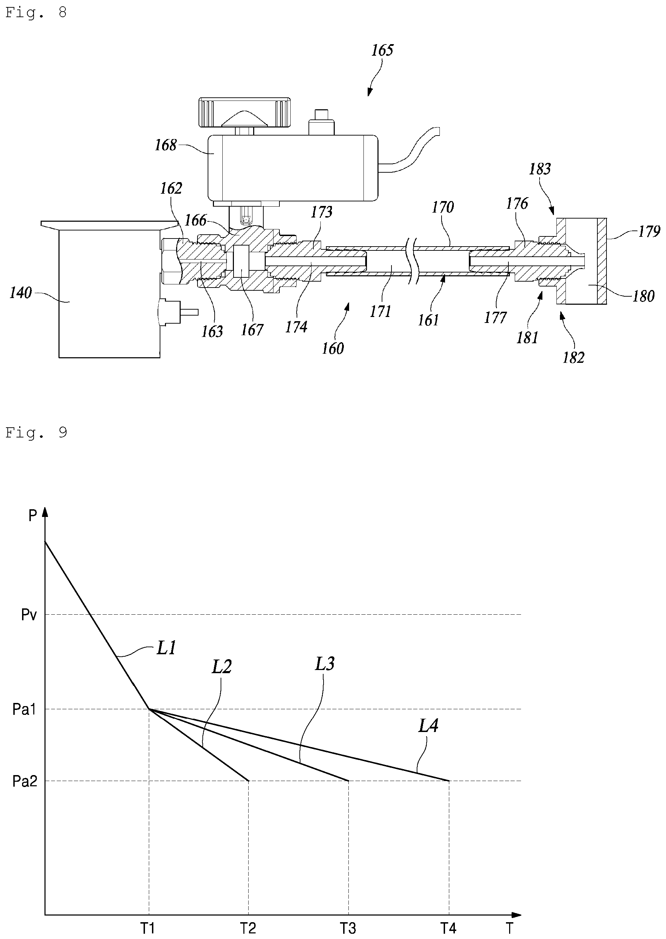

For example, the sterilization apparatus 100 according to the present invention may operate such that the internal pressure of the vaporizer 140 is varied in the procedure of concentrating the hydrogen peroxide solution, as in a graph shown in FIG. 9. In FIG. 9, a vertical axis represents an internal pressure P of the vaporizer 140, and a horizontal axis represents a time T for which the negative pressure is applied to the vaporizer 140.

In this case, the sterilization apparatus 100 may perform a process of concentrating the hydrogen peroxide solution such that the time for which the negative pressure is applied to the vaporizer 140 is varied to correspond to a multiple of a minimum reference time T1 in accordance with a predetermined condition.

In addition, the sterilization apparatus 100 may control an output of the vacuum pump 150 such that the internal pressure of the vaporizer 140 is linearly lowered along a reference pressure variation line L1 having a predetermined reference slope in the minimum reference time T1. In the procedure of lowering the internal pressure of the vaporizer 140 in the minimum reference time T1, the controller 190 may receive a measurement signal from the pressure gauge 145 generated through measurement of the internal pressure of the vaporizer 140, and may control an output of the vacuum pump 150 in order to prevent the internal pressure of the vaporizer 140 from exceeding a predetermined first target pressure pa1.

In addition, when the time for which the negative pressure is applied to the vaporizer 140 sequentially increases to times T2, T3, T4,... corresponding to n times the minimum reference time T1, the sterilization apparatus 100 may control an output of the vacuum pump 150 such that, in each time exceeding the minimum reference time T1, the internal pressure of the vaporizer 140 is lowered at a rate different from that of the minimum reference time T1.

In detail, in the minimum reference time T1, the sterilization apparatus 100 may control an output of the vacuum pump 150 such that the internal pressure of the vaporizer 140 is linearly lowered along the reference pressure variation line L1 having the reference slope and reaches the first target pressure pa1 when the minimum reference time T1 ends. A method of maintaining the output of the vacuum pump 150 at a predetermined first output may be used in order to linearly lower the internal pressure of the vaporizer 140 along the reference pressure variation line L1. The first target pressure pa1 may be set to a suitable pressure lower than an evaporation pressure Pv at which evaporation of moisture from the hydrogen peroxide solution starts.

In addition, after the minimum reference time T1 ends, the sterilization apparatus 100 may control an output of the vacuum pump 150 such that the internal pressure of the vaporizer 140 is linearly lowered along pressure variation lines L2, L3, L4,... having predetermined slopes and reaches a predetermined second target pressure pa2 when a time corresponding to n times the minimum reference time T1 ends. A method of maintaining the output of the vacuum pump 150 at a predetermined output may be used in order to linearly lower the internal pressure of the vaporizer 140 along the pressure variation lines L2, L3, L4,... having the predetermined slopes. The second target pressure pa2 may be set to a suitable pressure higher than the first target pressure pa1. For example, the second target pressure pa2 may be set to a pressure at which evaporation of hydrogen peroxide from the hydrogen peroxide solution starts or a pressure at which the amount of hydrogen peroxide evaporated from the hydrogen peroxide solution reaches a meaningful evaporation amount. The meaningful evaporation amount of hydrogen peroxide may be an amount corresponding to A% of a moisture evaporation amount, and A% may be set to various values such as 10%, 20%, 30%, etc.

For example, when the time for which the negative pressure is applied to the vaporizer 140 is a time T2 corresponding to 2 times the minimum reference time T1, as depicted in the graph of FIG. 9, the controller 190 may control the vacuum pump 150 such that the internal pressure of the vaporizer 140 is linearly lowered along the reference pressure variation line L1, until the minimum reference time T1 ends, and may then control the vacuum pump 150 such that the internal pressure of the vaporizer 140 is linearly lowered along a second pressure variation line L2, until the time T2 corresponding to 2 times the minimum reference time T1 ends after ending of the minimum reference time T1. A method of maintaining the output of the vacuum pump 150 at a predetermined second output may be used in order to linearly lower the internal pressure of the vaporizer 140 along the second pressure variation line L2.

In addition, when the time for which the negative pressure is applied to the vaporizer 140 is a time T3 corresponding to 3 times the minimum reference time T1, the controller 190 may control the vacuum pump 150 such that internal pressure of the vaporizer 140 is linearly lowered along the reference pressure variation line L1, until the minimum reference time T1 ends, and may then control the vacuum pump 150 such that the internal pressure of the vaporizer 140 is linearly lowered along a third pressure variation line L3, until the time T3 corresponding to 3 times the minimum reference time T1 ends after ending of the minimum reference time T1. A method of maintaining the output of the vacuum pump 150 at a predetermined third output may be used in order to linearly lower the internal pressure of the vaporizer 140 along the third pressure variation line L3.

As such, it may be possible to more rapidly remove moisture from the hydrogen peroxide solution, to minimize the amount of hydrogen peroxide discharged during removal of moisture and to reduce waste of hydrogen peroxide by controlling the sterilization apparatus 100 such that the lowering rate of the internal pressure of the vaporizer 140 is varied in accordance with the time for which the negative pressure is applied to the vaporizer 140, in the procedure of concentrating the hydrogen peroxide solution.

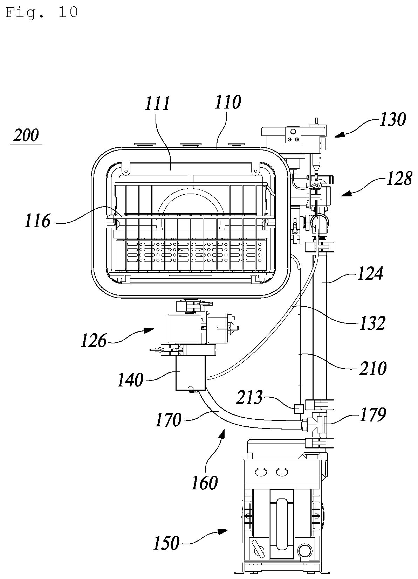

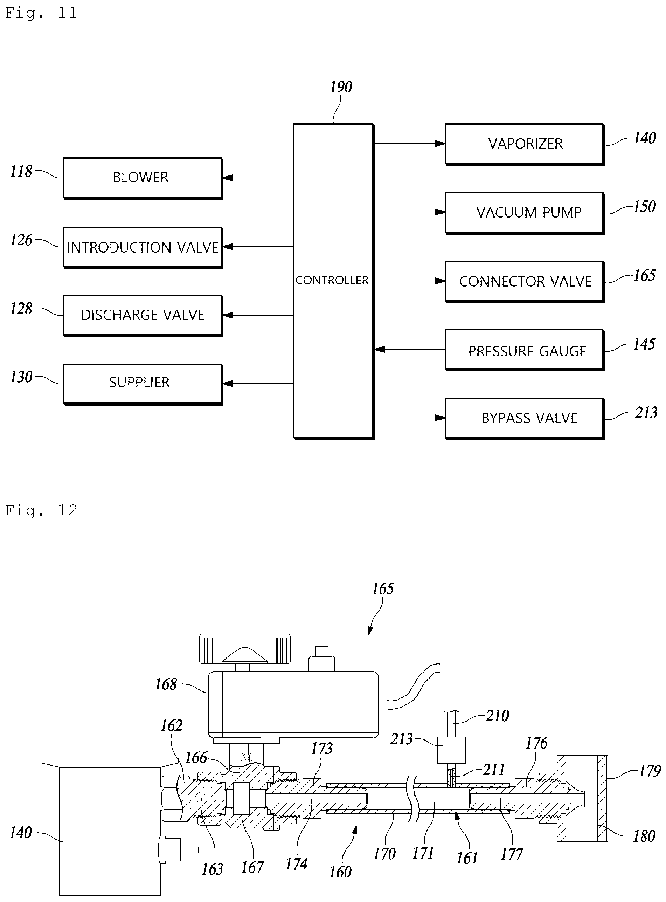

Meanwhile, FIG. 10 is a front view showing a sterilization apparatus according to another embodiment of the present invention. FIG. 11 is a block diagram showing a part of configurations of the sterilization apparatus according to the other embodiment of the present invention. FIG. 12 is a sectional view showing the vaporizer and the connector assembly in the sterilization apparatus according to the other embodiment of the present invention.

A sterilization apparatus 200 shown in FIGs. 10 to 12 further includes a bypass pipe 210, as compared to the above-described sterilization apparatus 100.

The bypass pipe 210 is configured to introduce fluid into the connecting flow path 161 of the connector assembly 160. The bypass pipe 210 interconnects the sterilization housing 110 and the connector assembly 160. A bypass pipe flow path 211 configured to flow gas from the sterilization chamber 111 to the connector pipe flow path 171 is provided within the bypass pipe 210.

A bypass valve 213 configured to open and close the bypass pipe flow path 211 is connected to the bypass pipe 210. The bypass valve 213 is controlled by the controller 190.

As in the above-described sterilization apparatus 100, the sterilization apparatus 200 according to the other embodiment of the present invention may sterilize an object, to be sterilized, in the sterilization chamber 111 by concentrating a hydrogen peroxide solution in the vaporizer 140, and introducing concentrated hydrogen peroxide into the sterilization chamber 111.

In a procedure of concentrating the hydrogen peroxide solution, the lowering velocity of the internal pressure of the vaporizer 140 may be varied due to various causes such as deformation of the connector assembly 160, etc.

When the lowering velocity of the internal pressure of the vaporizer 140 is higher than a predetermined velocity in the procedure of concentrating the hydrogen peroxide solution, the sterilization apparatus 200 according to the other embodiment of the present invention may decrease the lowering velocity of the internal pressure of the vaporizer 140 by introducing gas from the sterilization chamber 111 into the connecting flow path 161 through the bypass pipe flow path 211.

In detail, when the internal pressure of the vaporizer 140 is lowered at a lowering rate corresponding to a greater slope than a predetermined reference slope in the minimum reference time T1 during the procedure of concentrating the hydrogen peroxide solution, the sterilization apparatus 200 may control the bypass valve 213 such that fluid is introduced into the connecting flow path 161 through the bypass pipe 210. When gas from the sterilization chamber 111 is introduced into the connecting flow path 161 during discharge of gas from the vaporizer 140 into the connecting flow path 161 as the vacuum pump 150 operates, the level of the negative pressure applied to the vaporizer 140 is lowered and, as such, the lowering velocity of the internal pressure of the vaporizer 140 may be decreased. Accordingly, it may be possible to rapidly adjust the lowering rate of the internal pressure of the vaporizer 140 without precisely controlling an output of the vacuum pump 150.

In addition, when the internal pressure of the vaporizer 140 is lowered at a lowering rate corresponding to a greater slope than the predetermined reference slope after the minimum reference time T1 ends, during the procedure of concentrating the hydrogen peroxide solution, the sterilization apparatus 200 may decrease the lowering velocity of the internal pressure of the vaporizer 140 by controlling the bypass valve 213 such that fluid is introduced into the connecting flow path 161 through the bypass pipe 210.

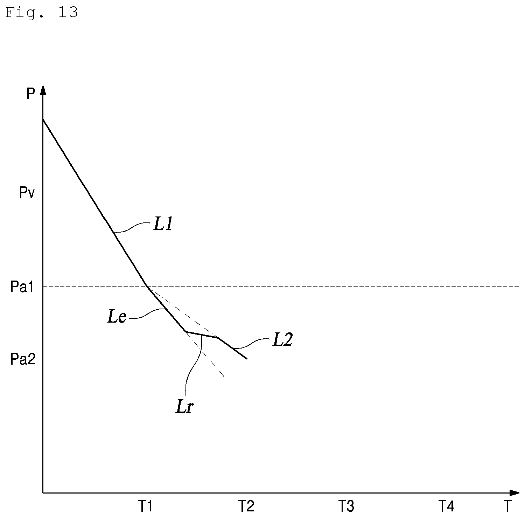

For example, the sterilization apparatus 200 according to this embodiment may operate such that the internal pressure of the vaporizer 140 is varied in the procedure of concentrating the hydrogen peroxide solution, as in a graph shown in FIG. 13.

When the time for which the negative pressure is applied to the vaporizer 140 is the time T2 corresponding to 2 times the minimum reference time T1, the controller 190 may control the vacuum pump 150 such that the internal pressure of the vaporizer 140 is linearly lowered along the reference pressure variation line L1, until the minimum reference time T1 ends, and may then control the vacuum pump 150 such that the internal pressure of the vaporizer 140 is linearly lowered along the second pressure variation line L2, until the time T2 corresponding to 2 times the minimum reference time T1 ends after ending of the minimum reference time T1.

Meanwhile, the lowering velocity of the internal pressure of the vaporizer 140 may be varied due to various causes such as a variation in flow resistance of gas through the connecting flow path 161, etc. under the condition that the output of the vacuum pump 150 is controlled in accordance with the process as described above.

For example, when the internal pressure of the vaporizer is lowered at a rate according to an abnormal pressure variation line Le having a greater slope than that of the second pressure variation line L2, in the time T2 corresponding to 2 times the minimum reference time T1, without varying along the second pressure variation line L2, the internal pressure of the vaporizer 140 may exceed the predetermined second target pressure pa2 when the time T2 corresponding to 2 times the minimum reference time T1 ends. In this case, there may be a problem in that an excessive amount of hydrogen peroxide evaporated from the hydrogen peroxide solution is discharged.

In order to eliminate such a problem, the sterilization apparatus 200 according to the other embodiment of the present invention may decrease the lowering velocity of the internal pressure of the vaporizer 140 when the internal pressure of the vaporizer 140 is lowered at a rate according to the abnormal pressure variation line Le, by controlling the bypass valve 213 such that fluid is introduced into the connecting flow path 161 through the bypass pipe 210. As fluid is introduced into the connecting flow path 161 through the bypass pipe 210, the internal pressure of the vaporizer 140 may be lowered at a rate according to an adjusted pressure variation line Lr.

As such, in a procedure in which the lowering rate of the internal pressure of the vaporizer 140 is adjusted, the controller 190 may enable introduction of a suitable amount of fluid into the connecting flow path 161 through the bypass pipe 210 by receiving a measurement signal for the internal pressure of the vaporizer 140 from the pressure gauge 145, and feedback-controlling the bypass valve 213 based on the measurement signal. In addition, it may be possible to rapidly adjust the lowering rate of the internal pressure of the vaporizer 140 such that the internal pressure of the vaporizer 140 is lowered at a rate according to the predetermined second pressure variation line L2, by introducing a suitable amount of fluid into the connecting flow path 161 through the bypass pipe 210.

Although the bypass pipe 210 is shown in the drawings as being configured to interconnect the sterilization housing 110 and the connector pipe 170, a concrete configuration of the bypass pipe 210 may be diversely changed. For example, the bypass pipe 210 may be configured to be connected to a part of the connector assembly 160 other than the connector pipe 170 in order to introduce fluid into the connecting flow path 161. In addition, although there is an advantage in terms of prevention of contamination of the connecting flow path 161 when gas from the sterilization chamber 111 is introduced into the connecting flow path 161 through the bypass pipe 210, the bypass pipe 210 may be configured to introduce air around the sterilization apparatus 100 into the connecting flow path 161 or may be configured to introduce other gas into the connecting flow path 161.

Although the embodiments of the present invention have been described with reference to the accompanying drawings, the present invention is not limited to such embodiments and may be implemented through various modifications without departing from the technical idea thereof. Accordingly, the embodiments disclosed in the present invention are not intended to limit the technical idea of the present invention, but to explain the present invention. The scope of technical idea of the present invention is not limited by the embodiments. Therefore, the embodiments described above should be understood as exemplary rather than limiting in all aspects. The scope of the present invention should be interpreted by the claims, and all technical ideas falling within a scope equivalent thereto should also be interpreted as falling within the scope of the present invention.

## Claims

1. A sterilization apparatus comprising:
a sterilization housing having a sterilization chamber configured to receive an object to be sterilized;
a vaporizer connected to the sterilization housing to supply hydrogen peroxide to the sterilization chamber;
a supplier configured to supply a hydrogen peroxide solution to the vaporizer;
a vacuum pump configured to apply a negative pressure to the vaporizer in order to lower an internal pressure of the vaporizer to an evaporation pressure enabling evaporation of moisture from the hydrogen peroxide solution;
a connector assembly comprising a connecting flow path configured to interconnect the vaporizer and the vacuum pump, and a connector valve configured to open and close the connecting flow path;
a bypass pipe having a bypass pipe flow path connected from the sterilization chamber to the connecting flow path;
a bypass valve configured to open and close the bypass pipe flow path; and
a controller configured to control the vacuum pump, the connector valve, and the bypass valve,
wherein, when a time for which the negative pressure is applied to the vaporizer is within a predetermined minimum reference time, an output of the vacuum pump is controlled such that the internal pressure of the vaporizer is linearly lowered at a lowering rate according to a predetermined reference slope, and reaches a predetermined first target pressure when the minimum reference time ends,
wherein, when the time for which the negative pressure is applied to the vaporizer corresponds to n times the predetermined minimum reference time, an output of the vacuum pump is controlled such that the internal pressure of the vaporizer is linearly lowered at a lowering rate according to a predetermined slope after the minimum reference time ends and reaches a predetermined second target pressure when a time corresponding to n times the minimum reference time ends,
wherein, when the internal pressure of the vaporizer is lowered at a lowering rate according to a greater slope than the reference slope in the minimum referent time, the bypass valve is controlled such that fluid is introduced into the connecting flow path through the bypass pipe, in order to adjust a lowering rate of the internal pressure of the vaporizer, and
wherein, when the internal pressure of the vaporizer is lowered at a lowering rate according to a greater slope than the predetermined slope after the minimum reference time ends, the bypass valve is controlled such that fluid is introduced into the connecting flow path through the bypass valve, in order to adjust the lowering rate of the internal pressure of the vaporizer.

2. The sterilization apparatus according to claim 1, wherein the connector assembly comprises:
a first end connector configured to interconnect the vaporizer and the connector valve while having a first end connector flow path;
a connector pipe disposed between the connector valve and the vacuum pump while having a connector pipe flow path;
a first middle connector connected to one end of the connector pipe to interconnect the connector valve and the connector pipe while having a first middle connector flow path;
a second middle connector connected to another end of the connector pipe while having a second middle connector flow path; and
a second end connector configured to interconnect the second middle connector and the vacuum pump while having a second end connector flow path.

3. The sterilization apparatus according to claim 2, wherein:
the first end connector flow path, a valve flow path of the connector valve, the first middle connector flow path, the connector pipe flow path, the second middle connector flow path, and the second end connector flow path form the connecting flow path; and
the connecting flow path has at least one diameter-increased section and at least one diameter-decreased section.

4. The sterilization apparatus according to claim 3, wherein the first end connector flow path is a smallest-diameter portion of the connecting flow path.

5. The sterilization apparatus according to claim 4, wherein:
a dimeter of the first middle connector flow path is equal to a diameter of the second middle connector flow path; and
a diameter of the connector pipe flow path is greater than the diameter of the first middle connector flow path and the diameter of the second middle connector flow path.

6. The sterilization apparatus according to claim 2, wherein the second end connector comprises:
a first connection portion connected to the second middle connector;
a second connection portion connected to the vacuum pump; and
a third connection portion connected to the sterilization housing.
